(19) Europäisches Patentamt
European Patent Office
Office européen des brevets

(11) Veröffentlichungsnummer: **0 068 369**
**B1**

(12) **EUROPÄISCHE PATENTSCHRIFT**

(45) Veröffentlichungstag der Patentschrift: **25.09.85**

(51) Int. Cl.⁴: **A 61 K 7/04**

(21) Anmeldenummer: **82105393.1**

(22) Anmeldetag: **19.06.82**

(54) **Nagelschutzpräparat zur Festigung, insbesondere Härtung, lebender Nägel.**

(30) Priorität: **26.06.81 CH 4233/81**

(43) Veröffentlichungstag der Anmeldung: **05.01.83 Patentblatt 83/1**

(45) Bekanntmachung des Hinweises auf die Patenterteilung: **25.09.85 Patentblatt 85/39**

(84) Benannte Vertragsstaaten: **DE FR GB IT**

(56) Entgegenhaltungen:
**DE - A - 1 767 165**
**FR - A - 1 569 578**
**GB - A - 1 301 904**
**US - A - 3 725 525**

(73) Patentinhaber: **Joos, Dr. Bernhard, Kurfirstenstrasse 23, CH-8002 Zürich (CH)**

(72) Erfinder: **Joos, Dr. Bernhard, Kurfirstenstrasse 23, CH-8002 Zürich (CH)**

(74) Vertreter: **Wann, Ingrid et al, Schmauder & Wann, Patentanwaltsbüro Nidelbadstrasse 75, CH-8038 Zürich (CH)**

EP 0 068 369 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Nagelschutzpräparat zur Festigung, insbesondere Härtung, lebender Nägel aus zwei Komponenten, von denen die erste eine polymerisierbare Verbindung und die zweite eine als Polymerisationskatalysator dienende Verbindung enthält.

Bekanntlich zeigen lebende Nägel, die aus Keratin aufgebaut sind, die Neigung, unter Einfluss von organischen Lösungsmitteln, wie sie beispielsweise in Nagellack und Nagellackentfernungsmitteln brüchig zu werden. Dieses Brüchigwerden führt zu einem Absplittern oder Einreissen der Nägel bei oft nur geringfügiger mechanischer Belastung.

Bei den bisher verwendeten Nagelschutzpräparaten wirkte es sich nachteilig aus, dass diese nur während eines verhältnismässig kurzen Zeitraums wirksam waren, da die gebildete Schutzschicht wasserlöslich war und somit beim Waschen sehr rasch ganz oder teilweise entfernt wurde. Ausserdem war bei einem Teil der bekannten Nagelschutzpräparate, sofern sie weniger löslich waren und die Bildung einer dichten Schicht bewirkten, die Gefahr des Erstickens der Nägel gegeben, wenn nicht durch sorgfältiges Auftragen verhindert wurde, dass sich die Schutzschicht bis zum Nagelbett hin erstreckte. So ist beispielsweise aus der US-Patentschrift 3 349 000 ein Verfahren zur Behandlung menschlicher Nägel und Haare bekannt, bei dem die Nägel bzw. Haare mit einer flüssigen Zubereitung, welche Dimethylolthioharnstoff in einer Menge von 1 bis 15 Gew.-%, bezogen auf das Gesamtgewicht der Zubereitung, und etwa 0,2 bis etwa 2 Gew.-% einer physiologisch unbedenklichen sauren Verbindung aus der aus Phosphorsäure, Milchsäure, Citronensäure, Essigsäure, Glycerinphosphorsäure und den sauren Salzen dieser Verbindungen bestehenden Gruppe sowie ein flüssiges Verdünnungsmittel enthält, behandelt werden. Aus der US-Patentschrift 3 725 525 ist ein Verfahren zum Festigen von lebenden menschlichen Nägeln und Haaren, bekannt, bei dem die Nägel oder Haare mit einer wirksamen Menge eines Dimethylolalkylenharnstoffs oder -thioharnstoffs der Formel

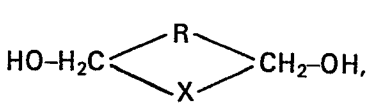

worin R eine Alkylengruppe mit 2 bis 5 Kohlenstoffatomen und X CO oder CS bedeuten, behandelt werden. Die mit Hilfe dieser bekannten Verfahren zu erzielenden Ergebnisse waren zwar besser als die mit den vorher bekannten Präparaten erhaltenen Ergebnisse, insbesondere hinsichtlich der Elastizität der behandelten Nägel, jedoch konnte auch mit Hilfe dieser Verfahren kein dauerhafter Schutz und insbesondere keine Härtung der Nägel erreicht werden. Ausserdem musste die Konzentration der Methylolgruppen aufweisenden Harnstoffderivate möglichst niedrig gehalten werden, um die Menge des bei der Reaktion freigesetzten Formaldehyds möglichst klein zu halten, da Formaldehyd bekanntlich Hautreizungen und insbesondere Allergien hervorruft. Um die schädliche Wirkung freiwerdenden Formaldehyds ganz oder zumindest grössten Teils auszuschalten, wurde in der US-Patentschrift 3 773 056, die Mittel und Verfahren zur Behandlung strapazierter Haare betrifft, vorgeschlagen, weniger leicht zersetzliche Harnstoff- bzw. Thioharnstoffderivate, wie Carbamate, Guanidine, Succinamide, Sulfonamide, Adipinamide oder Hydantoine, zu verwenden und diese mit organischen Säuren oder entsprechenden sauren Salze zur Reaktion zu bringen, indem man diese Säuren oder Salze vor, während oder nach der Behandlung mit dem Harnstoff- bzw. Thioharnstoffderivat auf das Haar aufbringt. Durch die in dieser US-Patentschrift beschriebenen Mittel und Verfahren wird ausschliesslich eine Festigung der Haare im Sinne einer Erhöhung der Elastizität keinesfalls jedoch eine Härtung bewirkt.

Aufgabe der Erfindung war es daher, ein Mittel zu schaffen, durch das eine dauerhafte Festigung, insbesondere Härtung, lebender Nägel erreicht wird und das frei von schädlich wirkenden Nebenprodukten, insbesondere von freiwerdenden Formaldehyd ist. Darüber hinaus sollte eine einfache und schnelle Handhabung gewährleistet sein, wobei vor allem angestrebt wurde, dass die Erzeugung der Schutzschicht in einem Arbeitsgang mit der Entfernung der Lackschicht bzw. vorhandener Lackreste vorgenommen werden kann.

Die Lösung dieser Aufgabe gelingt mit Hilfe des erfindungsgemässen Nagelschutzpräparates aus zwei getrennt aufzubewahrenden und nacheinander aufzutragenden Komponenten, von denen die erste eine polymerisierbare Verbindung und die zweite eine als Polymerisationskatalysator dienende Verbindung enthält, das dadurch gekennzeichnet ist, dass die erste Komponente als polymerisierbare Verbindung Dimethyloläthylenthioharnstoff in einer Menge von mindestens 18 Gew.-%, bezogen auf das Gesamtgewicht dieser Komponente, ein Purin, Wasser und ein mit Wasser mischbares organisches Lösungsmittel oder Lösungsmittelgemisch enthält und einen pH-Wert im Bereich von 8 bis 10 aufweist und dass die zweite Komponente als Polymerisationskatalysator 0,1 n bis 0,5 n Salzsäure in einer Menge, die ausreicht, um in dieser Komponente einen pH-Wert von 1 bis 3 aufrecht zu erhalten, sowie eine Aminosäure und als Lösungsmittel ein mit Wasser mischbares organisches Lösungsmittelgemisch enthält.

Der obere Grenzwert des Gehalts an Dimethyloläthylenthioharnstoff ergibt sich aus der Löslichkeit des Dimethyloläthylenthioharnstoffs in dem zur Verwendung kommenden Lösungsmittelgemisch, wobei allgemein ein Gehalt von etwa 18 Gew.-% bevorzugt ist. Als organisches Lösungsmittelgemisch in der Komponente 1 kommen bevorzugt solche Lösungsmittelgemische in Betracht, wie sie üblicherweise als Nagellackentfernungsmittel verwendet werden. Als besonders vorteilhaft hat sich ein Lösungsmittelgemisch aus Aceton/Essigsäureäthylester/Äthanol im Verhältnis 2:1:1 erwiesen.

Die Zugabe eines Purins zur Komponente 1 bewirkt eine zusätzliche Stabilisierung der Dimethyldoläthylenthioharnstofflösung. Als besonders geeignetes Purin ist Theophyllin zu nennen, welches vorzugsweise in einer Menge von 0,1 bis 0,2 Gew.-%, bezogen auf das Gesamtgewicht der Komponente 1, zugesetzt wird.

Die Zugabemenge der Salzsäure hängt von ihrem Verdünnungsgrad und vom pH-Wert der übrigen Bestandteile der Komponente 2 ab, wesentlich ist nur, dass in der Komponente 2 ein pH-Wert von 1 bis 3 eingestellt und aufrechterhalten wird. Auf diese Weise wird erreicht, dass die für eine rasche und vollständige Polymerisation des Dimethylolaethylenthioharnstoffs erforderliche Säuremenge vorhanden ist.

Als Aminosäuren eignen sich insbesondere niedermolekulare und einfach gebaute Aminosäuren, wobei Glycin besonders hervorzuheben ist. Versuche haben ergeben, dass beispielsweise als Aminosäure in der Komponente 2 auch Histidin verwendet werden kann. Die dabei erzielten Ergebnisse waren jedoch weniger überzeugend als die mit Glycin als Zusatz erhaltenen.

Bei Verwendung von Glycin als Aminosäure empfehlen sich Zugabemengen von 0,3 bis 1 Gew.-%, bezogen auf das Gesamtgewicht der Komponente, wobei Mengen von 0,78 bis 0,9 Gew.-% bezogen auf das Gesamtgewicht der Komponente 2, bevorzugt sind.

Als mit Wasser mischbare Lösungsmittel in Komponente 2 kommen beispielsweise Äthanol, Aceton oder deren Gemische sowie Lösungsmittelgemische wie sie in der Komponente 1 zur Anwendung kommen in Betracht. Besonders bevorzugt ist ein Lösungsmittelgemisch aus Aceton/Essigsäureäthylester/Äthanol im Verhältnis 2:1:1.

Ausserdem kann der Komponente 2 eine geringe Menge Dimethyloläthylenthioharnstoff, beispielsweise 0,02 Gew.-%, bezogen auf das Gesamtgewicht der Komponente, zugegeben werden.

Gemäss einer besonders bevorzugten Ausführungsform enthält die erste Komponente als Purin Theophillin und als organisches Lösungsmittelgemisch ein Gemisch aus Aceton/Essigsäureäthylenester/Äthanol im Verhältnis 2:1:1. Durch die Verwendung dieses Lösungsmittelgemisches, dessen Zusammensetzung derjenigen von üblicherweise verwendeten Nagellackentfernungsmitteln entspricht, wird erreicht, dass die Auftragung des Nagelschutzpräparates gleichzeitig mit der Entfernung vorhandener Lackreste vorgenommen werden kann, d.h. die erste Komponente des Nagelschutzpräparates dient gleichzeitig als Lackentfernungsmittel. Das erfindungsgemässe Nagelschutzpräparat wird vorzugsweise in Kombinationspackungen, welche die beiden Komponenten in getrennten Packungen, zum Beispiel Fläschchen, Phyolen oder Sachets, sowie gegebenenfalls noch geeignete Auftragseinrichtungen, z.B. Pinsel, Wattetupfer oder Vliestücher, enthalten, angeboten. Besonders zu empfehlen sind Kombinationspackungen aus zwei Sachets, die mit der Komponente 1 bzw. der Komponente 2 getränkte Vliestücher enthalten.

Mit derartigen Kombinationspackungen können die beiden Komponenten leicht nacheinander aufgetragen werden, wobei beim Auftragen der Komponente 1 bei geeigneter Wahl des Lösungsmittelgemisches vorhandene Lackrückstände gleichzeitig entfernt werden. Bei allen Arten von Verpackungen ist auf einen luftdichten Verschluss zu achten, um das Verdampfen der enthaltenen Lösungsmittel bzw. Lösungsmittelgemische zu verhindern.

Bei der Verwendung des erfindungsgemässen Nagelschutzpräparates geht man zweckmässigerweise so vor, dass man die Komponente 1 mit einem geeigneten Hilfsmittel auf den Nagel aufbringt und gegebenenfalls vorhandene Lackrückstände entfernt, die Komponente 1 während einer kurzen Wartezeit von einigen Minuten antrocknen lässt und danach anschliessend die Komponente 2, ebenfalls unter Verwendung geeigneter Hilfsmittel, aufträgt. Dieser Vorgang kann gewünschtenfalls ein-/oder mehrmals wiederholt werden. Nach einer verhältnismässig kurzen Trocknungszeit wird ein dünner, fester und wasserunlöslicher Schutzüberzug erhalten, der sich über die ganze Oberfläche des behandelten Nagels erstreckt. Da die Struktur des Schutzüberzuges, wie mikroskopische Untersuchungen gezeigt haben, netzartig ist, kommt es auch bei einer vollständigen Bedekkung der gesamten Nageloberfläche bis zum Nagelbett hin nicht zu einer Erstickung der behandelten Nägel.

Durch die Verwendung des erfindungsgemässen Nagelschutzpräparates wird nicht nur das Verspröden der Nägel durch Einwirkung von organischen Lösungsmitteln oder Wasch- und Reinigungsmittel verhindert, vielmehr zeigen die Nägel nach der Behandlung nicht nur subjektiv eine stark verbesserte Elastizität und insbesondere eine erheblich verbesserte Härte, sondern erweisen sich auch objektiv, wie durch Mikrohärte-Vergleichsmessungen festgestellt wurde, als erheblich härter als im Vergleich zu unbehandelten Nägel, worauf im folgenden noch näher eingegangen werden soll.

Da die durch Auftragen des erfindungsgemässen Nagelschutzpräparates erzeugte Schutzschicht, die durch eine Polymerisation der Dimethyloläthylenthioharnstoffmoleküle untereinander und durch Brückenbildung zwischen den Aminogruppen der Keratinmoleküle und den Methylolgruppen des Dimethylolthioharnstoffs zustande kommt, wasserunlöslich ist, wird ein dauerhafter Schutz erreicht, der auch nach längerer Zeit und nach wiederholtem Kontakt mit Lösungsmitteln, Wasch- und Reinigungsmitteln erhalten bleibt. Ausserdem kann die gebildete Schutzschicht als Grundierung für das Auftragen von Farblack dienen und ergibt eine haftfähige, glatte und glänzende Lackierung, die auch hohen ästhetischen Anforderungen genügt.

Die Erfindung soll nunmehr anhand einiger Ausführungsbeispiele, die besonders bevorzugte

Rezepturen enthalten und das Ergebnis von Mikrohärte-Vergleichsmessungen wiedergegeben näher erläutert werden.

Herstellung des Präparates

A: Lösung I (Komponente 1)

Die Herstellung der Lösung erfolgte durch inniges Vermischen der Komponenten unter kräftigem Rühren bei Raumtemperatur, wobei entweder der Dimethyloläthylenthioharnstoff und das Theophyllin gemeinsam zunächst in Wasser gelöst und anschliessend mit dem organischen Lösungsmittelgemisch vereinigt wurden oder der Dimethyloläthylenthioharnstoff im organischen Lösungsmittelgemisch und das Theophyllin in Wasser gelöst und anschliessend die beiden Lösungen vereinigt wurden. In beiden Fällen wurde eine leicht opuleszierende Lösung erhalten.

Beispiel 1 A

| | |
|---|---|
| Dimethyloläthylen-thioharnstoff (DMET) | 60 g (18,2 Gew.-%) |
| Theophyllin | 0,6 g |
| Wasser | 30 ml |
| Lösungsmittelgemisch * | 300 ml |
| pH-Wert | 9,6 |

* Aceton: Essigsäureäthylester:Äthanol = 2:1:1

Beispiel 2 A

| | |
|---|---|
| DMET | 62 g (18,8 Gew.-%) |
| Theophyllin | 0,6 g |
| Wasser | 30 ml |
| Lösungsmittelgemisch * | 300 ml |
| pH-Wert | 9,7 |

* Aceton: Essigsäureäthylester:Äthanol = 2:1:1

Beispiel 3 A

| | |
|---|---|
| DMET | 64 g (19,4 Gew.-%) |
| Theophyllin | 0,65 g |
| Wasser | 30 ml |
| Lösungsmittelgemisch * | 300 ml |
| pH-Wert | 9,8 |

* Aceton: Essigsäureäthylester:Äthanol = 2:1:1

Beispiel 4 A

| | |
|---|---|
| DMET | 67 g (20,3 Gew.-%) |
| Theophyllin | 0,7 g |
| Wasser | 30 ml |
| Lösungsmittelgemisch * | 300 ml |
| pH-Wert | 9,8 |

* Aceton: Essigsäureäthylester:Äthynol = 2:1:1

B: Lösung II (Komponente 2)

Die Herstellung erfolgte durch Lösen der Aminosäure in Wasser oder verdünnter Salzsäure und anschliessendes Vermischen dieser Lösung mit dem organischen Lösungsmittel bzw. Lösungsmittelgemisch. Auch in diesem Fall wurde bei Raumtemperatur gearbeitet und durch kräftiges Rühren für eine gute Durchmischung gesorgt.

Beispiel 1 B:

| | |
|---|---|
| Glycin | 10 g |
| Wasser | 50 ml |
| HCl (0,5n) | 210 ml |
| Lösungsmittelgemisch * | 1000 ml |
| pH-Wert | 2,8–3 |

* Aceton: Essigsäureäthylester: Äthanol = 2:1:1

Beispiel 2 B

| | |
|---|---|
| Glycin | 20 g |
| Wasser | 100 ml |
| HCl (0,5 n) | 420 ml |
| Lösungsmittelgemisch * | 2000 ml |
| Rapsöl | 0,0005 Gew.-% |
| pH-Wert | 2,8 |

* Aceton: Essigsäureäthylester:Äthanol = 2:1:1

Beispiel 3 B

| | |
|---|---|
| Glycin | 2,0 g |
| HCl (0,1 n) | 250 ml |
| Äthanol/Aceton (1 : 1) | 100 ml |
| pH-Wert | 2,0 |

Beispiel 4 B

| | |
|---|---|
| Glycin | 3,3 g |
| HCl (0,1 n) | 760 ml |
| Äthanol/Aceton (1 : 1) | 240 ml |
| DMET | 0,1 g |
| pH-Wert | 1,3 |

Prüfung der Schutzwirkung für Keratin

A: Lebende Nägel

Zur Durchführung des Versuches wurden Fingernägel innerhalb von 4 Minuten jeweils 1 bis 3 mal mit einer Lösung I, deren Zusammensetzung einer der in den Beispielen 1A bis 4A angegebenen entsprach, behandelt, wobei die Lösung mit Hilfe eines Wattetupfers aufgetragen wurde. Nach einer Wartezeit von 1 bis 3 Minuten wurde dann eine Lösung II, deren Zusammensetzung einer der in den Beispielen 1B bis 4B angegebenen entsprach, aufgetragen. Nach einer Trocknungszeit von 3 bis 6 Minuten, wobei das Trocknen durch mit Hilfe eines Föhns erzeugte Warmluft beschleunigt werden konnte, hatte sich ein fester, polierfähiger Überzug gebildet, der wasserunlöslich war und mit einem zugespitzten Federkiel nicht mehr greitzt werden konnte. Dabei zeigte es sich, dass die Bildung des festen Überzuges umso rascher von statten ging, je niedriger der pH-Wert der Lösung II war. Dies deutet darauf hin, dass die Polymerisation des Dimethyloläthylenthioharnstoffs umso schneller und vollständiger verläuft, je höher der Gehalt der Lösung II an Säure ist.

B: Indikatorversuch an Federkielen

Federkiel-Abschnitte wurden nacheinander mit Lösungen I und II, deren Zusammensetzungen den in den Beispielen 1A bis 4A bzw. 1B bis 4B angegebenen entsprachen, behandelt, wobei das Auftragen der Lösungen durch Tauchen erfolgt.

Bei einem Teil der Federkiel-Abschnitte wurde die Behandlung einmal oder mehrmals wiederholt, wonach die behandelten Federkiel-Abschnitte zusammen mit unbehandelten in Ninhydrin-Lösung eingebracht wurden. Das Eintreten der Farbreaktion wurde beobachtet und die Zeit bis zum Eintreten bestimmt. Dabei ergab sich folgendes Bild:

| Zahl der Behandlungen | 1 | 2 | 3 | unbehandelt |
|---|---|---|---|---|
| Farbreaktion * | ++ | + | – | +++ |
| Zeit bis zum Eintreten der Farbreaktion (Min.) | 3 | 4 | 10 | 2 |

| | | |
|---|---|---|
| * Farbreaktion | negativ | – |
| Farbreaktion | sehr schwach positiv | + |
| Farbreaktion | schwach positiv | ++ |
| Farbreaktion | stark positiv | +++ |

Dieser Versuch zeigte, dass bereits durch eine einmalige Behandlung eine Schutzschicht erzeugt wird, die bewirkt, dass nur eine schwach positive Farbreaktion auftritt, während sich bei den unbehandelten Proben schon nach 2 Minuten eine stark positive Farbreaktion zeigte. Bei der 2× behandelten Probe war erst nach 4 Minuten eine sehr schwach positive Reaktion feststellbar, während bei der 3× behandelten Probe auch nach 10 Minuten keine Reaktion festzustellen war. Auch nach weiteren 60 Minuten konnte keine Farbreaktion wahrgenommen werden. Dies zeigte, dass bereits nach einmaliger Behandlung eine Schutzschicht vorhanden war, durch die zwar die Reaktion des Keratin mit dem Ninhydrin nicht vollständig unterbunden, jedoch stark behindert wurde. Durch Wiederholung der Behandlung wurde die Schutzschicht verstärkt, so dass schliesslich zwischen dem Keratin und dem Ninhydrin keine Reaktion mehr stattfand.

C: Mikrohärte-Messungen

Die Messungen wurden mit Hilfe eines Kleinlast-Härteprüfers «Durimet» durchgeführt. Die Prüflast betrug 200 g, die Dauer der Belastung 15 Sekunden. Als Messgrösse wurde die Diagonallänge der Eindrücke in µm bei 100-facher Vergrösserung bestimmt. Als Probekörper wurden in Kunstharzblöcke eingegossene Keratinplättchen aus Schweinehufen verwendet. Die Keratinplättchen wurden nacheinander mit einer Lösung I und dann mit einer Lösung II behandelt, wobei die Lösungen mit Hilfe eines mit der jeweiligen Lösung getränkten Papiervlieses aufgetragen wurden. Die verwendeten Lösungen I und II waren nach den in den Beispielen 1A bis 4A bzw. 1B bis 4B angegebenen Rezepturen hergestellt worden.

Die Ergebnisse dieser Messungen sind in der nachfolgenden Tabelle aufgeführt. Die bei den einzelnen Messungen verwendeten Lösungen sind durch die Nummern der Beispiele, in denen die Zusammensetzungen angegeben sind, bezeichnet.

Tabelle

| Probe Nr. | Lösung I | Wartezeit )* (Min.) | Lösung II | Trocknung (Min.) | Temp. (°C) | Diagonallänge )** Mittelwert (µm) | Diagonallänge )** Mittelwert (µm) | Differenz (µm) |
|---|---|---|---|---|---|---|---|---|
| 1A/1B | 1× | 2 | 1× | 3 | 25 | 35,2 | 33,9 | 1,9 |
| | 3× | 3 | 3× | 5 | 25 | 35,2 | 32,2 | 3,0 |
| 1A/2B | 1× | 2 | 1× | 3 | 25 | 35,4 | 32,8 | 2,6 |
| | 3× | 3 | 3× | 5 | 25 | 35,4 | 32,1 | 3,3 |
| 1A/3B | 1× | 2 | 1× | 3 | 25 | 35,4 | 33,1 | 2,3 |
| | 3× | 3 | 3× | 5 | 25 | 35,4 | 32,0 | 3,4 |
| 1A/4B | 1× | 2 | 1× | 3 | 25 | 35,5 | 32,9 | 2,6 |
| | 3× | 3 | 3× | 5 | 25 | 35,5 | 31,8 | 3,7 |
| 2A/1B | 1× | 2 | 1× | 3 | 25 | 34,6 | 33,2 | 1,4 |
| | 3× | 3 | 3× | 5 | 25 | 35,9 | 33,5 | 2,4 |
| 2A/2B | 1× | 2 | 1× | 3 | 25 | 37,1 | 35,3 | 1,8 |
| | 3× | 3 | 3× | 5 | 25 | 35,8 | 32,6 | 3,2 |
| 2A/3B | 1× | 2 | 1× | 3 | 25 | 37,0 | 33,8 | 3,2 |
| | 3× | 3 | 3× | 5 | 25 | 36,5 | 32,2 | 4,3 |
| 2A/4B | 1× | 2 | 1× | 3 | 25 | 37,0 | 33,6 | 3,4 |
| | 3× | 3 | 3× | 5 | 25 | 36,2 | 32,2 | 4,0 |
| 3A/1B | 1× | 2 | 1× | 3 | 25 | 35,9 | 33,5 | 2,4 |
| | 3× | 3 | 3× | 5 | 25 | 35,9 | 32,2 | 3,7 |
| 3A/2B | 1× | 2 | 1× | 3 | 25 | 35,8 | 33,2 | 2,6 |
| | 3× | 3 | 3× | 5 | 25 | 35,8 | 32,0 | 3,8 |
| 3A/3B | 1× | 2 | 1× | 3 | 25 | 35,6 | 33,1 | 2,5 |
| | 3× | 3 | 3× | 5 | 25 | 35,6 | 32,0 | 3,6 |
| 3A/4B | 1× | 2 | 1× | 3 | 25 | 35,5 | 32,8 | 2,7 |
| | 3× | 3 | 3× | 5 | 25 | 35,5 | 31,6 | 3,9 |
| 4A/1B | 1× | 2 | 1× | 3 | 25 | 35,6 | 33,2 | 2,4 |
| | 3× | 3 | 3× | 5 | 25 | 35,6 | 32,2 | 3,6 |
| 4A/2B | 1× | 2 | 1× | 3 | 25 | 36,7 | 33,9 | 2,8 |
| | 3× | 3 | 3× | 5 | 25 | 36,3 | 32,6 | 3,7 |
| 4A/3B | 1× | 2 | 1× | 3 | 25 | 36,2 | 32,8 | 3,4 |
| | 3× | 3 | 3× | 3 | 50 | 36,3 | 32,2 | 4,1 |
| 4A/4B | 1× | 2 | 1× | 3 | 25 | 37,0 | 33,6 | 3,4 |
| | 3× | 3 | 3× | 3 | 50 | 36,5 | 32,5 | 4,0 |

* Wartezeit zwischen den Behandlungen mit Lösung I und Lösung II
** Diagonallänge des Eindrucks bei 100-facher Vergrösserung

**Patentansprüche**

1. Nagelschutzpräparat zur Festigung, insbesondere Härtung, lebender Nägel aus zwei getrennt aufzubewahrenden und nacheinander aufzutragenden Komponenten, von denen die erste eine polymerisierbare Verbindung und die zweite eine als Polymerisationskatalysator dienende Verbindung enthält, dadurch gekennzeichnet, dass die erste Komponente als polymerisierbare Verbindung Dimethyloläthylenthioharnstoff in eine Menge von mindestens 18 Gew.-%, bezogen auf das Gesamtgewicht dieser Komponente, ein Purin, Wasser und ein mit Wasser mischbares organisches Lösungsmittelgemisch enthält und einen pH-Wert im Bereich von 8 bis 10 aufweist und dass die zweite Komponente als Polymerisationskatalysator 0,1 n bis 0,5 n Salzsäure in eine Menge, die ausreicht, um in dieser Komponente einen pH-Wert von 1 bis 3 aufrechtzuerhalten, sowie eine Aminosäure und als Lösungsmittel ein mit Wasser mischbares organisches Lösungsmittel oder Lösungsmittelgemisch enthält.

2. Nagelschutzpräparat nach Patentanspruch 1, dadurch gekennzeichnet, dass das mit Wasser mischbare organische Lösungsmittelgemisch in der ersten Komponente ein Gemisch aus Aceton, Essigsäureäthylester und Äthanol im Verhältnis 2:1:1 ist.

3. Nagelschutzpräparat nach Patentanspruch 2, dadurch gekennzeichnet, dass die zweite Komponente ebenfalls ein Gemisch aus Aceton, Essigsäureäthylester und Äthanol im Verhältnis 2:1:1 als mit Wasser mischbares organisches Lösungsmittelgemisch enthält.

4. Nagelschutzpräparat nach Patentanspruch 1, dadurch gekennzeichnet, dass das Purin Theophyllin ist und dass dieses in eine Menge von 0,1 bis 0,2 Gew.-%, bezogen auf das Gesamtgewicht der ersten Komponente, vorhanden ist.

5. Nagelschutzpräparat nach Patentanspruch 1, dadurch gekennzeichnet, dass die Aminosäure Glycin ist und dass dieses in einer Menge von 0,3 bis 1 Gew.-%, bezogen auf das Gesamtgewicht der zweiten Komponente, vorhanden ist.

6. Nagelschutzpräparat nach Patentanspruch 1, dadurch gekennzeichnet, dass die erste Komponente einen pH-Wert von 9,6 aufweist und die folgende Zusammensetzung hat:

| | |
|---|---|
| – Dimethyloläthylenthioharnstoff | 60 g |
| – Theophyllin | 0,6 g |
| – Wasser | 30 ml |
| – Gemisch aus Aceton, Essigsäureäthylester und Äthanol (2:1:1) | 300 ml |

und dass die zweite Komponente einen pH-Wert von 2,8 bis 3 aufweist und die folgende Zusammensetzung hat:

| | |
|---|---|
| – Glycin | 10 g |
| – Wasser | 50 ml |
| – 0,5 n HCl | 210 ml |
| – Gemisch aus Aceton, Essigsäureäthylester und Äthanol (2:1:1) | 1000 ml |

7. Nagelschutzpräparat nach Patentanspruch 1, dadurch gekennzeichnet, dass die erste Komponente die in Patentanspruch 6 angegebene Zusammensetzung hat und dass die zweite Komponente einen pH-Wert von 2,0 aufweist und die folgende Zusammensetzung aufweist:

| | |
|---|---|
| – Glycin | 0,2 g |
| – 0,1 n HCl | 25 ml |
| – Äthanol | 5 ml |
| – Aceton | 5 ml |

8. Nagelschutzpräparat nach Patentanspruch 1, dadurch gekennzeichnet, dass die erste Komponente die in Patentanspruch 6 angegebene Zusammensetzung hat und dass die zweite Komponente einen pH-Wert von 1,3 aufweist und die folgende Zusammensetzung hat:

| | |
|---|---|
| – Glycin | 3,33 g |
| – 0,1 n HCl | 760 ml |
| – Äthanol | 120 ml |
| – Aceton | 120 ml |
| – Dimethyloläthylenthioharnstoff | 0,15 g |

9. Nagelschutzpräparat nach einem der Patentansprüche 1 bis 8, dadurch gekennzeichnet, dass es in Form einer Kombinationspackung aus zwei luftdicht verschlossenen Sachets vorliegt, von denen das eine ein mit der ersten Komponente getränktes Vliestuch und das andere ein mit der zweiten Komponente getränktes Vliestuch enthält.

10. Nagelschutzpräparat nach einem der Patentansprüche 1 bis 8, dadurch gekennzeichnet, dass es in Form einer Kombinationspackung aus zwei luftdicht verschlossenen Fläschchen, die vorzugsweise mit einer Auftragvorrichtung ausgestattet sind, vorliegt, wobei das eine Fläschchen die erste Komponente und das andere die zweite Komponente enthält.

11. Verwendung des Nagelschutzpräparates nach Patentanspruch 1 zum Festigen und insbesondere Härten lebender Finger- und Zehennägel, dadurch gekennzeichnet, dass man die erste Komponente unter gleichzeitiger Entfernung vorhandener Lackrückstände dünn und gleichmässig auf die Nägel aufträgt und die so gebildete Schicht antrocknen lässt und dass man anschliessend die zweite Komponente in einer Menge, die zur Bildung einer dünnen, den ganzen Nagel bedeckenden Schutzschicht ausreicht, aufträgt und die so behandelten Nägel trocknen lässt, bis sich eine festhaftende, gegen mechanische Belastung beständige und in Wasser und organischen Lösungsmitteln unlösliche Schutzschicht gebildet hat.

**Revendications**

1. Préparation pour la protection des ongles vivants, destinée à leur renforcement, en particulier leur durcissement, faite de deux composants que l'on doit conserver séparément et appliquer successivement, dont le premier contient un composé polymérisable et le second, un composé servant de catalyseur pour la polymérisation, caractérisée

en ce que le premier composant contient, comme composé polymérisable, de la diméthyloléthylènethiourée, dans une proportion d'au moins 18% en poids, calculé sur le poids total de ce composant, une purine, de l'eau, et un mélange de solvants organiques miscibles à l'eau, et a un pH d'une valeur de l'ordre de 8 à 10, et ce que le second composant contient, comme catalyseur de polymérisation de l'acide chlorhydrique 0,1 à 0,5 n, dans une proportion qui suffit pour établir dans ce composant un pH d'une valeur de 1 à 3, ainsi qu'un acide aminé, et, comme solvant, un solvant organique ou un mélange de solvants organiques miscibles à l'eau.

2. Préparation pour la protection des ongles suivant la revendication 1, caractérisée en ce que le mélange de solvants organiques miscible à l'eau du premier composant est un mélange d'acétone, d'acétate d'éthyle et d'éthanol dans le rapport de 2:1:1.

3. Préparation pour la protection des ongles, suivant la revendication 2, caractérisée en ce que le second composant contient également un mélange d'acétone, d'acétate d'éthyle et d'éthanol dans le rapport de 2:1:1, comme mélange de solvants organiques miscible à l'eau.

4. Préparation pour la protection des ongles suivant la revendication 1, caractérisée en ce que la purine est de la théophylline, et que celle-ci est présente dans une proportion de 0,1 à 0,2% en poids, calculé sur le poids total du premier composant.

5. Préparation pour la protection des ongles suivant la revendication 1, caractérisée en ce que l'acide aminé est la glycine, et que celle-ci est présente dans une proportion de 0,3 à 1% en poids, calculé sur le poids total du second composant.

6. Préparation pour la protection des ongles suivant la revendication 1, caractérisé en ce que le premier composant présente un pH de 9,6 et a la composition suivante:

| | |
|---|---|
| – diméthyloléthylènethiourée | 60 g |
| – théophylline | 0,6 g |
| – eau | 30 ml |
| – mélange d'acétone, d'acétate d'éthyle et d'éthanol (2:1:1) | 300 ml |

et que le second composant présente un pH de 2,8 à 3 et a la composition suivante:

| | |
|---|---|
| – glycine | 10 g |
| – eau | 50 ml |
| – HCl 0,5 n | 210 ml |
| – mélange d'acétone, d'acétate d'éthyle et d'éthanol (2:1:1) | 1000 ml |

7. Préparation pour la protection des ongles suivant la revendication 1, caractérisée en ce que le premier composant a la composition indiquée dans la revendication 6, et que le second composant présente un pH de 2, et a la composition suivante:

| | |
|---|---|
| – glycine | 0,2 g |
| – HCl 0,1 n | 25 ml |
| – éthanol | 5 ml |
| – acétone | 5 ml |

8. Préparation pour la protection des ongles suivant la revendication 1, caractérisée en ce que le premier composant a la composition indiquée dans la revendication 6, et que le second composant présente un pH de 1,3 et a la composition suivante:

| | |
|---|---|
| – glycine | 3,33 g |
| – HCl 0,1 n | 760 ml |
| – éthanol | 120 ml |
| – acétone | 120 ml |
| – diéméthyloléthylènethiourée | 0,15 g |

9. Préparation suivant l'une des revendications 1 à 8, caractérisée en ce qu'elle se présente sous la forme d'un emballage combiné fait de deux sachets fermés, étanches à l'air, dont l'un contient un tissu de nappe de fibres imprégné du premier composant et l'autre, un tissu de nappe de fibres imprégné du second composant.

10. Préparation suivant l'une des revendications 1 à 8, caractérisée en ce qu'elle se présente sous la forme d'un emballage combiné fait de deux flacons fermés étanches à l'air, qui sont pourvus de préférence d'un instrument d'application, l'un des flacons contenant le premier composant, et l'autre, le second composant.

11. Utilisation de la préparation pour la protection des ongles suivant la revendication 1, pour rendre plus solides et en particulier pour durcir les ongles vivants des doigts et des orteils, caractérisée en ce que l'on applique le premier composant sur l'ongle, en éliminant en même temps les résidus de vernis qui pourraient s'y trouver, en une couche mince et uniforme et laisse sécher la couche ainsi formée, et que l'on applique ensuite le second composant en une quantité qui suffise pour la formation d'une couche protectrice mince recouvrant toute la surface de l'ongle, et laisse sécher l'ongle ainsi traité, jusqu'à ce qu'il se soit formé une couche résistante aux contraintes mécaniques et insoluble dans l'eau et les solvants organiques.

## Claims

1. Preparation for improving strength, particularly hardness, of living human nails comprising two separate components which have to be stored separately and to be applied successively wherein the first component contains a polymerizable compound and the second component contains a polymerization catalyst, characterized in that the first component contains, as a polymerizable compound, at least 18 percent by weight of the total weight of this component dimethylol ethylene thiourea, a purine, water, and a water-miscible organic solvent, and has a pH value ranging from 8 to 10; and that the second compound contains, as a polymerization catalyst, 0,1 n to 0,5 n hydrochloric acid in a quantity sufficent to maintain a pH value ranging from 1 to 3 in this component as well as an amino acid, and a water-miscible organic solvent or solvent mixture.

2. Preparation according to patent claim 1,

characterized in that the water-miscible organic solvent mixture used in the first component is a mixture of acetone, ethyl acetate, and aethanol in the ration 2 to 1 to 1.

3. Preparation according to patent claim 2, characterized in that the second component also contains, as a water-miscible organic solvent mixture, a mixture of acetone, ethyl acetate, and aethanol in the ration 2 to 1 to 1.

4. Preparation according to patent claim 1, characterized in that the purine is theophylline and is present in a quantity of 0,1 to 0,2 percent by weight of the total weight of the first component.

5. Preparation according to patent claim 1, characterized in that the amino acid is amino acetic acid and is present in a quantity of 0,3 to 1 percent by weight of the total weight of the second component.

6. Preparation according to patent claim 1, characterized in that the first component has a pH value of about 9,6 and is composed as follows:

| | |
|---|---|
| – dimethylol ethylene thiourea | 60 g |
| – theophylline | 0,6 g |
| – water | 30 ml |
| – mixture of acetone, ethyl acetate and ethanol (2:1:1) | 300 ml |

and the second component has a pH value of about 2,8 to 3 and is composed as follows:

| | |
|---|---|
| – amino acetic acid | 10 g |
| – water | 50 ml |
| – 0,5 n hydrochloric acid | 210 ml |
| – mixture of acetone, ethyl acetate and ethanol (2:1:1) | 1000 ml |

7. Preparation according to patent claim 1, characterized in that the first component is composed as indicated in patent claim 6 and that the second component has a pH value of about 2,0 and is composed as follows:

| | |
|---|---|
| – amino acetic | 0,2 g |
| – 0,1 n hydrochloric acid | 25 ml |
| – ethanol | 5 ml |
| – acetone | 5 ml |

8. Preparation according to patent claim 1, characterized in that the first component is composed as indicated in patent claim 6 and that the second component has a pH value of about 1,3 and is composed as follows:

| | |
|---|---|
| – amino acetic acid | 3,33 g |
| – 0,1 n hydrochloric acid | 760 ml |
| – ethanol | 120 ml |
| – acetone | 120 ml |
| – dimethylol ethylene thiourea | 0,15 g |

9. Preparation according to one of patent claims 1 to 8, characterized in that the preparation is present in the form of a combination package composed of two hermetically sealed sachets; one of said sachets containing a nonwoven fabric impregnated with the first component and the other of said sachets containing a non-woven fabric impregnated with the second component.

10. Preparation according to one of patent claims 1 to 8, characterized in that the preparation is present in the form of a combination package composed of two hermetically sealed flasks which are preferably provided with an applicator; on of said flasks containing the first component and the other containing the second component.

11. Application of the preparation of patent claim 1 for improving strength, particularly hardness, of living human nails, characterized in that the first component is applied thinly and uniformly to the nails simultaneously removing any present residues of nail polish, whereupon the formed coating is allowed to dry, then the second component is applied to the nails in a quantity sufficient to cover the whole nail with a thin protective coating, whereupon the such treated nails are allowed to dry until a firmly adherent protective coating insoluble in water and organic solvents and resistant to mechanical stress has been formed.